# EUROPEAN PATENT APPLICATION

(11) **EP 3 235 497 A1**
(43) Date of publication of application: **25.10.2017**
(21) Application number: 15851742.5
(22) Date of filing: 23.10.2015
(51) Int. Cl.: A61K 31/137, A61P 35/00

(54) **USES OF CINACALCET HCL IN PREPARING PHARMACEUTICAL COMPOSITION FOR TREATMENT OF CANCER**

(30) Priority: 24.10.2014 US 201462068298 P
(71) Applicant: Launx Biomedical Co., Ltd., Kaohsiung City 801 (TW)
(72) Inventor: CHEN, Chiu-Hung, Kaohsiung City 801 (TW); CHUANG, Show-Mei, Taichung City 408 (TW); WAY, Tzong-Der, Kaohsiung City 801 (TW); HSIAO, Nai-Wan, Taichung City 402 (TW)
(74) Representative: Schwerbrock, Florian
(86) International application number: PCT/CN2015/092776
(87) International publication number: WO 2016/062286

(57) **Abstract**

The present invention provides a new clinical indication of Cinacalcet HCl. Cinacalcet HCl is already approved by FDA. The present invention provides a Cinacalcet HCl pharmaceutical composition for treating a variety of cancer cells effectively.

## Description

### CROSS-REFERENCE TO RELATED APPLICATIONS

This is a National Phase Application filed under 35 U.S.C. 371 as a national stage of PCT/CN2015/092776 filed Oct. 23, 2015, an application claiming the benefit of U.S. Provisional Applications No. 62/068,298 filed Oct. 24, 2014, the content of each of which is hereby incorporated by reference in its entirety.

### FIELD OF THE INVENTION

The present invention related to a new indication of Cinacalcet hydrochloride (Cinacalcet HCl) pharmaceutical composition, especially related to inhibition effect of Cinacalcet hydrochloride (Cinacalcet HCl) pharmaceutical composition on a variety of cancer cells.

### BACKGROUND OF THE INVENTION

Cancer is the most popular disease cause of death in the world. The cancer patients are gradually increase yearly, therefore the treatment method of the cancer has become an important issue. The medical treatments of cancer can be classified as surgical treatment, radiation therapy, chemotherapy and target therapy.

Generally, the cancer drug, whether chemotherapy drug or target therapy drug, is to inhibit cancer cells duplication and split to prevent the tumor growth and metastasis.

More attention of therapies is being focused on drugs or other substances that block the growth and spread of cancer by interfering with specific molecules ("molecular targets") that are involved in the growth, progression, and spread of cancer. Averagely, only about five of 10,000 new drugs can successfully enter the phase I of clinical trials.

Otherwise, the manufacturing of the drug is still a big problem. When the drug starting the clinical trials, there are lots of problems need to overcome, such as drug safety, patient selection, trial dose and other issues. Even the drug has approved by the FDA and sales on the market, there still possibly face the situation of the poor drug response in patients. Furthermore, if the cancer patients happen the drug resistance, that would reduce the effectiveness of the drugs and result in the medical treatment failure. Therefore, the new drug development is very difficult.

Cinacalcet hydrochloride (Cinacalcet HCl) or calcimimetic is a naphthalene derivative and calcimimetic agent that increases the sensitivity of parathyroid gland calcium-sensing receptors to serum calcium. (Cinacalcet HCl) is the orally bioavailable hydrochloride salt of the calcimimetic cinacalcet. Cinacalcet increases the sensitivity of calcium-sensing receptors on chief cells in the parathyroid gland to extracellular calcium, thereby reducing parathyroid hormone (PTH) secretion. In other word, these actions could reduce parathyroid hormone secretion and decreases serum calcium in the treatment of parathyroid disease. Further, a reduction in PTH levels inhibits osteoclast activity, which may result in a decrease in cortical bone turnover and bone fibrosis, and normalization of serum calcium and phosphorus levels. In another way, Cinacalcet HCl therapy was used for secondary hyperparathyroidism in hemodialysis patients. As a result, Cinacalcet HCl is approved by FDA and accumulated a huge data of drug use and drug mechanism research.

Due to the differences of the clinical use, there is no research present that the Cinacalcet HCl has any potential to inhibit cancer cell.

### SUMMARY OF THE INVENTION

In order to solve the above problems, the present invention provides the development of new cancer clinical indications of Cinacalcet HCl.

Accordingly, the present invention provides a new indication of Cinacalcet HCl. The experimental results showed that the Cinacalcet HCl had no toxicity or had little toxicity to normal cells in the present invention. However, the selective effect of Cinacalcet HCl between normal cells and cancer cells need to be identified.

The present invention provides a pharmaceutical composition of Cinacalcet HCl for treating cancer. The pharmaceutical composition is composed of effective dose of Cinacalcet HCl and a pharmaceutical acceptable salt.

In one embodiment of the present invention, the cancer is selected from pleural-related cancer, abdominal-related cancer, endocrine-related cancer, gastrointestinal tract-related cancer.

In one embodiment of the present invention, the cancer is selected from osteosarcoma, skin cancer and blood cancer.

In one embodiment of the present invention, the pleural-related cancer is lung cancer.

In one embodiment of the present invention, the abdominal-related cancer is selected from bladder cancer, and cervical cancer.

In one embodiment of the present invention, the endocrine-related cancer is selected from prostate cancer, breast cancer, and ovarian cancer.

In one embodiment of the present invention, the gastrointestinal tract-related cancer is selected from gastric cancer, hepatic cancer, colorectal cancer, pancreatic cancer, and tongue cancer.

In one embodiment of the present invention, the effective dose of Cinacalcet HCl is from 10 mg/kg/day to 500 mg/kg/day.

### BRIEF DESCRIPTION OF THE DRAWINGS

FIG 1 shows the inhibitory effect of Cinacalcet HCl on the different cancer cells.
FIG 2 shows the results of the inhibitory effect of tumor volume by Cinacalcet HCl.
FIG 3 shows the inhibitory effect of tumor growth via administered high-dose and low-dose of Cinacalcet HCl.

### DETAILED DESCRIPTION OF THE INVENTION

### Cell culture

Subculture the different types of cancer cells. The cancer cells includes lung cancer, gastric cancer, hepatic cancer, colon cancer, skin cancer, cervical cancer, prostate cancer, bladder cancer, breast cancer, leukemia, pancreatic cancer, ovarian cancer, tongue cancer, osteosarcoma, and renal cancer. The normal cells used in the control group included kidney cell (HEK293) and human bronchial epithelial cell line BEAS-2B (as shown in Table 1).

Cancer cell lines were cultured in different culture medium according to different characteristics (as shown in Table 1). The cell numbers were counted and reseed as 2×10⁶ in culture plate/flask. Then, the culture medium were added to a volume of 10 ml, and the cells were cultured for 2-3 days. Then, the cells were suspended for loading into 96-well plates. The number of cells was 3000 and the volume of the culture medium was 100 µl each well.

**Table 1. Cancer cell lines and culture medium**

| No | Cancer type | Cancer cell type | Culture medium |
|---|---|---|---|
| 1 | lung cancer | H1650 (lung adenocarcinoma) | RPMI-1640 |
| | | A549 (lung adenocarcinoma) | DMEM |
| 2 | gastric cancer | AGS (Gastric Adenocarcinoma) | RPMI-1640 |
| | | MKN-45 (Gastric Adenocarcinoma) | RPMI-1640 |
| 3 | hepatic cancer | HepG2 (hepatocellular carcinoma) | DMEM |
| | | Hep3B (hepatocellular carcinoma) | DMEM |
| 4 | colon cancer | HCT116(p53+) (colorectal carcinoma) | DMEM |
| | | LoVo(Colorectal Adenocarcinoma) | DMEM |
| 5 | skin cancer | A375 (amelanotic melanoma) | DMEM |
| | | BCC (basal cell carcinoma) | DMEM |
| 6 | cervical cancer | HeLa (Cervix Adenocarcinoma) | DMEM |
| | | C-33A (Cervical carcinoma) BCRC60554 | MEM |
| 7 | prostate cancer | PC3 (p53-)(Prostate adenocarcinoma) | DMEM |
| | | LNCaP clone FGC (LNCap.FGC) | RPMI-1640 |
| 8 | bladder cancer | 8301 (urinary bladder carcinoma) | RPMI-1640 |
| | | T24 | RPMI-1640 |
| 9 | breast cancer | MCF7 (Mammary Gland, Adenocarcinoma) | DMEM |
| | | MDA-MB-231 (Mammary Gland, Adenocarcinoma) | DMEM |
| 10 | pancreatic cancer | BxPC-3 | RPMI-1640 |
| | | AsPC-1 | RPMI-1640 |
| 11 | ovarian cancer | NIH:OVCAR-3 | RPMI-1640 |
| | | TOV-21G | RPMI-1640 |
| 12 | tongue cancer | SAS (Tongue squamous cell carcinoma) | DMEM |
| 13 | osteosarcoma | U-2OS | DMEM |
| 14 | renal cancer | 786-0 (Renal adenocarcinoma) BCRC 60243 | RPMI-1640 |
| 15 | normal cell | | |
| | kidney | HEK293 (Kidney) | DMEM |
| | pulmonary epithelial cell line | BEAS-2B *(Lung* Epithelial*)* | RPMI-1640 |

### Cell viability analysis

Removing the original culture medium from 96-well plate. Then add 100 µl of commercially drug at a concentration of 10 µM per well. After 72 hours, add the diluted WST-1 reagent to the well with 100 µl/well, and the diluted WST-1 reagent was acquired from the dilution of 9:1 medium and WST-1 stock reagent. Finally, the total volume of each well was 200 µl/well. Culture the 96-well plate at 37 °C for 30 to 90 minutes. Detecting and calculate the survival rate of each cancer cells with an ELISA reader at OD450nm. The lower viability of cancer cells represents better inhibition effect via the Cinacalcet HCl drug. Otherwise, the higher viability of cancer cells represents worse inhibition effect via the Cinacalcet HCl drug.

### The effect of Cinacalcet HCl on different cancer cell lines

### The inhibition effect of Cinacalcet HCl on pleural-related cancer cells

This inhibition test of Cinacalcet HCl on pleural-related cancer cells were using two lung cancer cell lines A549 and H1650. The inhibitory tests of Cinacalcet HCl were performed 4 times for each cell lines and then the average value of the inhibitory tests was calculated. The results were shown in Table 2.

**Table 2. The inhibition effect of Cinacalcet HCl on pleural-related cancer cells**

| | 0524-10 min | 0526-10 min | 0529-10 min | 0531-10 min | Average |
|---|---|---|---|---|---|
| A549 | 87.7 | 102.7 | 75.5 | 92.6 | 89.6 |

| | 1-10 min | 2-20 min | 3-20 min | 4-20 min | Average |
|---|---|---|---|---|---|
| H1650 | 86.8 | 67.5 | 76.8 | 75.2 | 76.5 |

### The inhibition effect of Cinacalcet HCl on abdominal-related cancer cell lines

This inhibition test of Cinacalcet HCl on abdominal-related cancer cells were using bladder cancer cell line TSGH and T24 (Table 3), cervical cancer cell lines HeLa and C-33A (Table 4), renal cancer cell line 786-O (Table 5). The inhibitory tests of Cinacalcet HCl were performed 4 times for each cell lines and then the average value of the inhibitory tests was calculated. The results were shown in Table 3, Table 4, and Table 5.

**Table 3. The inhibition effect of Cinacalcet HCl on bladder cancer cell lines**

| | 0510-10 min | 0512-10 min | 0515-10 min | 0517-10 min | average |
|---|---|---|---|---|---|
| TSGH | 57.3 | 73.2 | 74.7 | 33.9 | 59.8 |

| | T24-1-30 min | T24-2-20 min | T24-3-20 min | T24-4-20 min | average |
|---|---|---|---|---|---|
| T24 | 71.1 | 132.6 | 177.1 | 103.3 | 121.0 |

**Table 4. The inhibition effect of Cinacalcet HCl on cervical cancer cell lines**

| | 0524-10 min | 0526-10 min | 0529-10 min | 0531-10 min | average |
|---|---|---|---|---|---|
| HeLa | 109.6 | 118.6 | 104.1 | 113.0 | 111.3 |
| C-33A | 114.5 | 118.8 | 108.1 | 100.0 | 110.3 |

**Table 5. The inhibition effect of Cinacalcet HCl on renal cancer cell lines**

| | 0524-10 min | 0526-10 min | 0529-10 min | 0531-10 min | average |
|---|---|---|---|---|---|
| 786-O | 81.3 | 71.6 | 80.2 | 87.1 | 80.0 |

### The inhibition effect of Cinacalcet HCl on endocrine-related cancer cell lines

This inhibition test of Cinacalcet HCl on endocrine-related cancer cells were using prostate cancer cell lines PC-3 and LNCap (Table 6), breast cancer cell lines MCF7 and MDA-MB-231 (Table 7), and ovarian cancer cell lines NIH-OVCAR-3 and TOV-21G (Table 8). The inhibitory tests of Cinacalcet HCl were performed 4 times for each cell lines and then the average value of the inhibitory tests was calculated. The results were shown in Table 6, Table 7, and Table 8.

**Table 6. The inhibition effect of Cinacalcet HCl on prostate cancer cell lines**

| | PC-3-0524-10 min | PC-3-0526-10 min | PC-3-0529-10 min | PC-3-0531-10 min | average |
|---|---|---|---|---|---|
| PC-3 | 102.7 | 132.2 | 120.3 | 134.6 | 122.5 |

| | Average | | | | |
|---|---|---|---|---|---|
| LNCap | 52.6 | | | | |

**Table 7. The inhibition effect of Cinacalcet HCl on breast cancer cell lines**

| | 0612-10 min | 0614-10 min | 0616-10 min | 0619-10 min | average |
|---|---|---|---|---|---|
| MCF7 | 87.7 | 75.6 | 82.2 | 77.2 | 80.7 |

| | 0612-10 min | 0614-10 min | 0616-10 min | 0619-10 min | average |
|---|---|---|---|---|---|
| MDA-MB-231 | 92.35 | 84.94 | 60.81 | 92.75 | 82.71 |

**Table 8. The inhibition effect of Cinacalcet HCl on ovarian cancer cell lines**

| | 7-3-30 min | 7-4-30 min | 7-7-30 min | -4-30 min | average |
|---|---|---|---|---|---|
| NIH-OVCAR-3 | 90.7 | 111.1 | 100.2 | 111.0 | 103.3 |

| | 7-3-30 min | 7-4-30 min | 7-7-30 min | -4-30 min | average |
|---|---|---|---|---|---|
| TOV-21G | 128.7 | 109.4 | 104.1 | 108.2 | 112.6 |

### The inhibition effect of Cinacalcet HCl on gastrointestinal tract-related cancer cell lines

This inhibition test of Cinacalcet HCl on gastrointestinal tract-related cancer cells were using gastric cancer cell lines AGS and MKN-45 (Table 9), hepatic cancer cell lines HepG2 and Hep3B (Table 10), colorectal cancer cell lines HCT116-wt and LoVo (Table 11), pancreatic cancer cell line AsPC-1 and BxPC-3(Table 12), tongue cancer cell line SAS (Table 13). The inhibitory tests of Cinacalcet HCl were performed 4 times for each cell lines and then the average value of the inhibitory tests was calculated. The results were shown in Table 9, Table 10, Table 11, Table 12 and Table 13.

**Table 9. The inhibition effect of Cinacalcet HCl on gastric cancer cell lines**

| | 0510-10 min | 0512-10 min | 0515-10 min | 0517-10 min | average |
|---|---|---|---|---|---|
| AGS | 26.6 | 20.0 | 52.3 | 14.50856943 | 28.3 |

| | 0510-10 min | 0512-10 min | 0515-10 min | 0517-10 min | average |
|---|---|---|---|---|---|
| MKN-45 | 82.1 | 101.5 | 103.6 | 69.1 | 89.1 |

**Table 10. The inhibition effect of Cinacalcet HCl on hepatic cancer cell lines**

| | 0524-20 min | 0526-20 min | 0529-20 min | 0531-20 min | average |
|---|---|---|---|---|---|
| HepG2 | 105.7 | 132.6 | 116.5 | 89.5 | 111.1 |

| | 0612-20 min | 0614-20 min | 0616-20 min | 0619-20 min | average |
|---|---|---|---|---|---|
| Hep3B | 143.2 | 125.1 | 151.5 | 116.8 | 134.1 |

**Table 11. The inhibition effect of Cinacalcet HCl on colorectal cancer cell lines**

| | 0602-30 min | 0605-10 min | 0607-10 min | 0609-10 min | average |
|---|---|---|---|---|---|
| HCT116-wt | 89.0 | 107.3 | 132.5 | 112.6 | 110.3 |

| | 0616-10 min | 0619-10 min | 0621-10 min | 0623-10 min | average |
|---|---|---|---|---|---|
| LoVo | 73.9 | 125.0 | 90.4 | 84.2 | 93.4 |

**Table 12. The inhibition effect of Cinacalcet HCl on pancreatic cancer cell lines**

| | 1-7-3-30 min | 1-7-4-30 min | 1-7-7-30 min | 1-4-30 min | Average |
|---|---|---|---|---|---|
| AsPC-1 | 61.6 | 81.7 | 60.4 | 71.9 | 68.9 |

| | 3-7-3-30 min | 3-7-4-30 min | 3-7-7-30 min | 3-4-30 min | Average |
|---|---|---|---|---|---|
| BxPC-3 | 64.6 | 78.8 | 100.1 | 76.2 | 79.9 |

**Table 13. The inhibition effect of Cinacalcet HCl on tongue cancer cell lines**

| | 6-26-10 min | 6-28-10 min | 6-30-10 min | 7-3-10 min | average |
|---|---|---|---|---|---|
| SAS | 45.7 | 88.9 | 96.2 | 98.9 | 82.4 |

### The inhibition effect of Cinacalcet HCl on other cancer cell lines

This inhibition test of Cinacalcet HCl on other cancer cells were using osteosarcoma cell line U2OS (Table 14), skin cancer cell lines A375 and BCC (Table 15). The inhibitory tests of Cinacalcet HCl were performed 4 times for each cell lines and then the average value of the inhibitory tests was calculated. The results were shown in Table 14 and Table 15.

**Table 14. The inhibition effect of Cinacalcet HCl on osteosarcoma cancer cell line**

| | 6-26-10 min | 6-28-10 min | 6-30-10 min | 7-3-10 min | average |
|---|---|---|---|---|---|
| U2OS | 82.6 | 86.7 | 64.9 | 75.3 | 77.4 |

**Table 15. The inhibition effect of Cinacalcet HCl on skin cancer cell lines**

| | 0602-30 min | 0605-10 min | 0607-10 min | 0609-10 min | average |
|---|---|---|---|---|---|
| A375 | 117.1 | 95.6 | 94.4 | 108.5 | 103.9 |
| | 0602-30 min | 0605-10 min | 0607-10 min | average | |
| BCC | 114.2 | 105.5 | 112.6 | 110.8 | |

### The experiment design on Control group

### The inhibition effect of Cinacalcet HCl on normal cells

This inhibition test of Cinacalcet HCl on normal cells were using normal kidney cell line HEK293 (Table 16), normal pulmonary epithelial cell lines canine fibroblast cell line BEAS-2B (Table 17). The inhibitory tests of Cinacalcet HCl were performed 4 times for each cell lines and then the average value of the inhibitory tests was calculated The results were shown in Tables 16 and Tables 17.

**Table 16. The inhibition effect of Cinacalcet HCl on normal kidney cell line**

| | average |
|---|---|
| HEK293 | 114.1 |

**Table 17. The inhibition effect of Cinacalcet HCl on normal pulmonary epithelial cell line**

| | 0510-10 min | 0512-10 min | 0515-10 min | 0517-10 min | average |
|---|---|---|---|---|---|
| BEAS-2B | 70.5 | 72.9 | 72.2 | 89.0 | 76.1 |

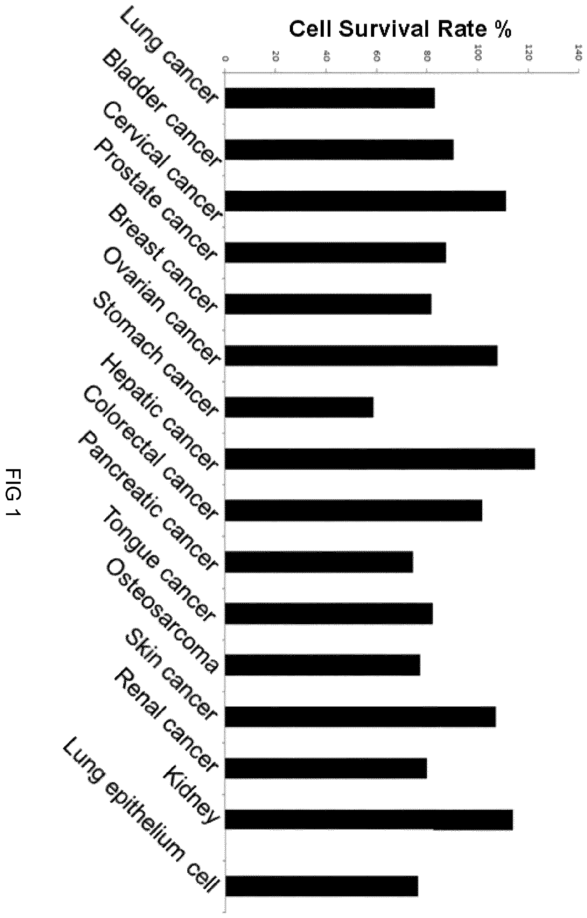

This inhibition test results of Cinacalcet HCl on all kinds of cells were shown in Table 18. Cinacalcet HCl is having different inhibitory effect on different tumor cell type even in the same cancer. As a result in the experiments of the present invention, Cinacalcet HCl has a significant inhibitory effect and specificity on various cancer cells. (FIG 1)

**Table 18. Summary of the Effect on different cancer cell lines by Cinacalcet HCl**

| cancer cells | Inhibitory effect |
|---|---|
| lung cancer | 83.09384194 |
| bladder cancer | 90.4 |
| cervical cancer | 111.32 |
| Kidney cancer | 87.53 |
| prostate cancer | 81.70 |
| breast cancer | 107.9 |
| ovarian cancer | 58.7 |
| gastric cancer | 122.60 |
| hepatic cancer | 101.85 |
| colorectal cancer | 74.4 |
| pancreatic cancer | 82.44 |
| tongue cancer | 77.37 |
| osteosarcoma | 107.32 |
| skin cancer | 80.0 |
| kidney cell lines | 114.06 |
| normal pulmonary epithelial cell line | 76.13 |

### Animal model test of gastric cancer with dose 100 mg/kg/day and 200 mg/kg/day

In this invention, the female mice were(BALB / cAnN.Cg-Foxn1^{nu} / CrlNarl) and purchased from National Laboratory Animal Center (Taiwan). The weight of the mice were 21 ± 1 g. These mice were subcutaneously injected with gastric cancer cells (AGS) and then put these mice into different cages at random. The drug test experiment was divided into three groups, include "control group", "low dose group (100 mg/kg/day)", "high dose group (200 mg/kg/day)". These mice were then injected test drug intraperitoneally once daily until the tumor size reached 100 mm³. The tumor sizes and body weight were measured twice a week. The tumor sizes were measured and calculated by formula: (L × W²)/2. L represents the tumor longest length. W represents the tumor shortest diameter. The experiment result is shown in Table 19.

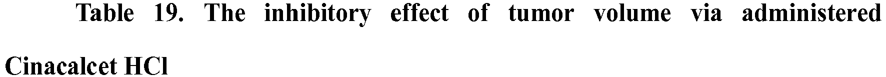

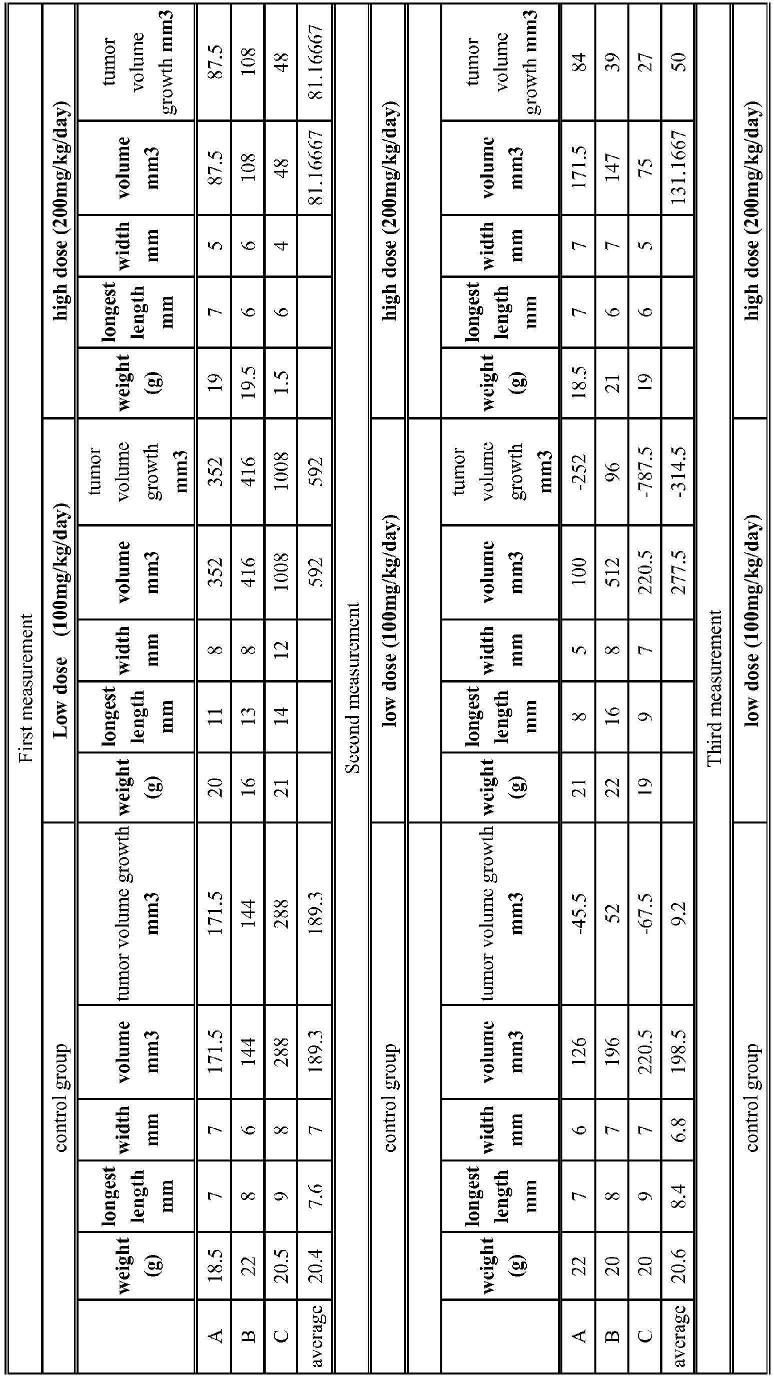

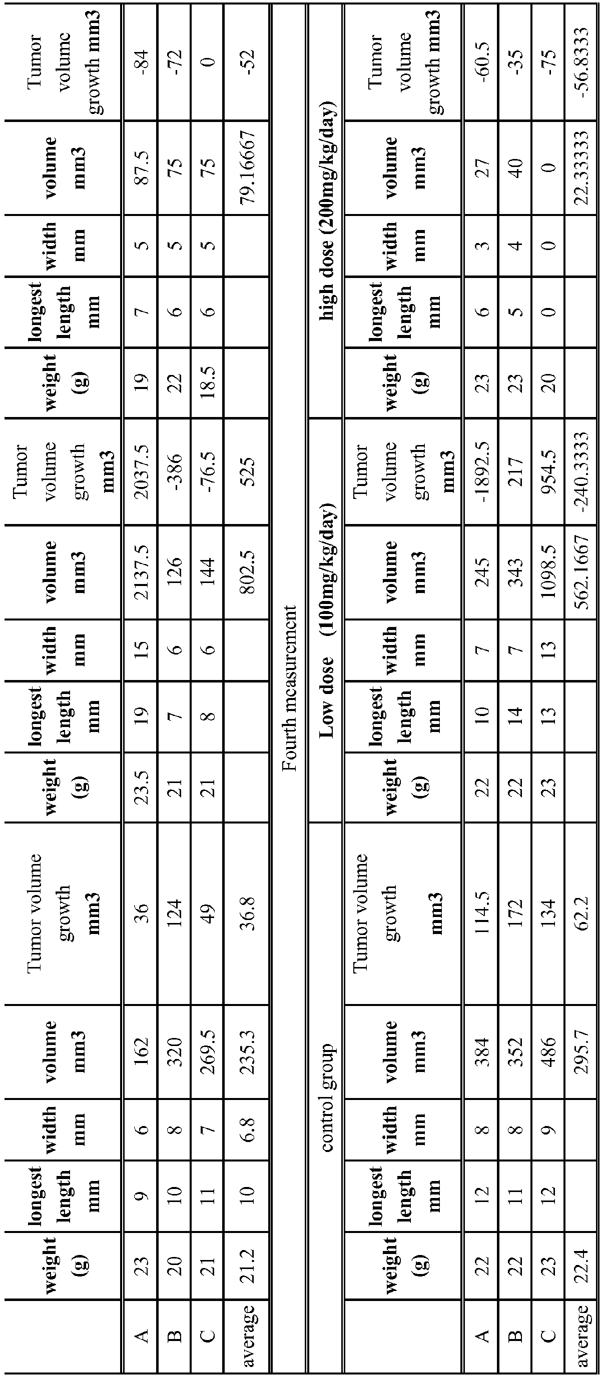

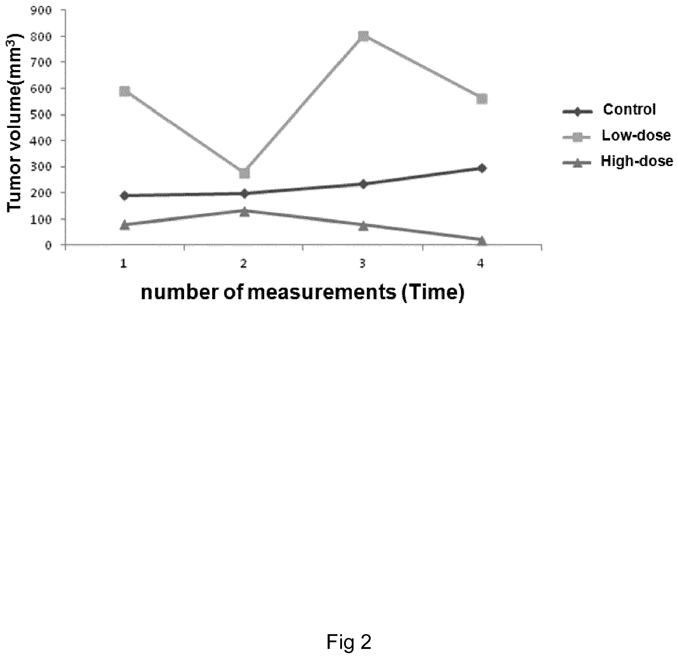

According to the results in FIG 2, both low dose and high dose of Cinacalcet HCl had significant inhibition effect on tumor cells, and the weight of mice did not show a significant decrease during the experiment. These results indicated that both high and low doses of Cinacalcet HCl could keep the tested mice in healthy status during the treatment without death.

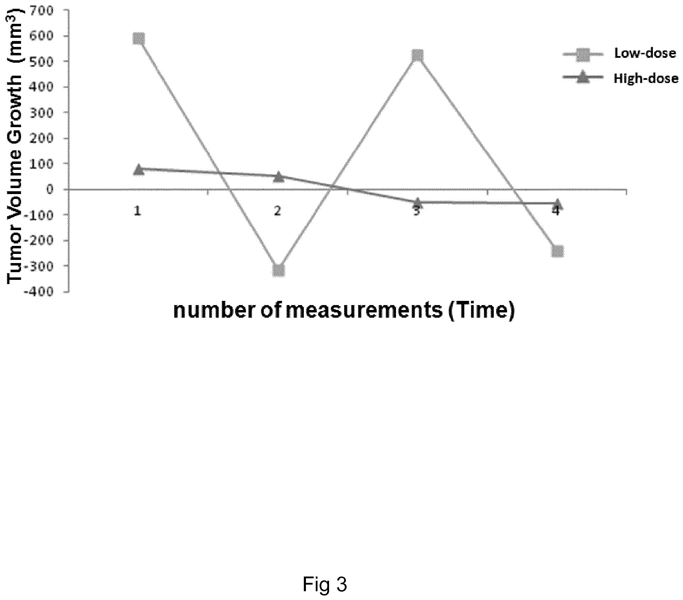

According to the results in FIG 3, both low dose and high dose of Cinacalcet HCl had effectively slow down the tumor volume growth, and can also reduce the tumor volume. Especially, high doses of Cinacalcet HCl had better effect to inhibit tumor growth.

Although the present invention has been described in terms of specific exemplary embodiments and examples, it will be appreciated that the embodiments disclosed wherein are for illustrative purposes only and various modifications and alterations might be made by those skilled in the art without departing from the spirit and scope of the invention as set forth in the following claims.

## Claims

1. A pharmaceutical composition for treating a cancer comprising a therapeutically effective amount of Cinacalcet HCl or a pharmaceutical acceptable salt thereof.

2. The pharmaceutical composition of claim 1, wherein the cancer is selected from pleural-related cancer, abdominal-related cancer, endocrine-related cancer and gastrointestinal tract-related cancer.

3. The pharmaceutical composition of claim 1, wherein the cancer is selected from osteosarcoma, skin cancer and blood cancer.

4. The pharmaceutical composition of claim 2, wherein the pleural-related cancer is lung cancer.

5. The pharmaceutical composition of claim 2, wherein the abdominal-related cancer is selected from bladder cancer, cervical cancer, and kidney cancer.

6. The pharmaceutical composition of claim 2, wherein the endocrine-related cancer is selected from prostate cancer, breast cancer, and ovarian cancer.

7. The pharmaceutical composition of claim 2, wherein the gastrointestinal tract-related cancer is selected from gastric cancer, hepatic cancer, colorectal cancer, pancreatic cancer, and tongue cancer.

8. The pharmaceutical composition of claim 1, wherein the effective amount of Cinacalcet HCl is from 20 mg/kg/day to 500 mg/kg/day.
